# EUROPEAN PATENT SPECIFICATION

(11) **EP 4 151 742 B1**
(45) Date of publication and mention of the grant of the patent: **10.01.2024**
(21) Application number: 21797925.1
(22) Date of filing: 30.07.2021
(51) Int. Cl.: C12P 19/02, C12P 13/00, C12P 7/40, C12P 7/24, C12P 19/26, C12N 15/70, C12N 5/10, C12N 1/21, C12N 9/78, C12N 9/12, C12R 1/19, C12R 1/125

(54) **TRANSGENIC CELL LINE AND GENETICALLY ENGINEERED BACTERIUM EXPRESSING FRUCTOSAMINE DEGLYCASE, AND USE OF FRUCTOSAMINE DEGLYCASE**
TRANSGENE ZELLLINIE UND GENMANIPULIERTE BAKTERIEN, DIE FRUCTOSAMIN-DEGLYKANASE EXPRIMIEREN, SOWIE VERWENDUNG VON FRUCTOSAMIN-DEGLYKANASE
LIGNÉE CELLULAIRE TRANSGÉNIQUE ET BACTÉRIE GÉNÉTIQUEMENT MODIFIÉE EXPRIMANT LA FRUCTOSAMINE DÉGLYCASE, ET UTILISATION DE LA FRUCTOSAMINE DÉGLYCASE

(43) Date of publication of application: 22.03.2023
(73) Proprietor: WUHAN BAI'ANHUIJI BIOTECHNOLOGY CO., LTD, Wuhan, Hubei 430074 (CN)
(72) Inventor: WANG, Huaiying, Wuhan, Hubei 430074 (CN)
(74) Representative: Kolster Oy Ab
(86) International application number: PCT/CN2021/109679
(87) International publication number: WO 2023/004772

(56) References cited:
- CN-A- 110 079 488
- CN-A- 112 852 774
- CN-A- 112 852 774
- CN-A- 113 046 401
- CN-A- 113 046 401
- US-A- 5 387 109
- US-A- 5 387 109
- DATABASE Protein [Online] 1 November 2016 (2016-11-01), Anonymous: "Fructosamine deglycase FrlB [Bacillus subtilis]", XP055954458, retrieved from Genbank Database accession no. AOR99552
- DATABASE Genbank [Online] 25 October 2013 (2013-10-25), ANONYMOUS: "ESD97983 fructokinase [Escherichia coli 908658]", XP009538610,
- DATABASE Protein 1 November 2016 (2016-11-01), Anonymous: "Fructosamine deglycase FrlB [Bacillus subtilis]", XP055954458, retrieved from Genbank Database accession no. AOR99552
- DATABASE GenBank 25 October 2013 (2013-10-25), Anonymous: "ESD97983 fructokinase [Escherichia coli 908658]", XP009538610, retrieved from NCBI Database accession no. ESD97983

## Description

### TECHNICAL FIELD

The disclosure relates to the technical field of production by a biological enzyme method, in particular to a fructosamine deglycase vector, a transgenic cell line and a genetic engineering bacteria expressing fructosamine deglycase, and use of fructosamine deglycase.

### BACKGROUND ART

Glucosamine (GlcN), i.e. 2-amino-2-deoxy-D-glucose, has important physiological functions for human body, and is widely used in food, health care products, medicines and other fields. In medical clinic, it can be used for treating bone and joint diseases, as a drug for treating rheumatoid arthritis, or as an additive in food for infants and young children. With the further improvement of the quality of life and the aggravation of population aging, the global demand for glucosamine medicines and food nutrition and health products will continue to increase.

There are two traditional methods for preparing glucosamine. One is to hydrolyze chitin. However, this process is restricted by seasonal influence of raw materials, complexity of hydrolysis process, large amount of acid and alkali, and environmental pollution. The other is to produce N- acetylglucosamine by microbial fermentation, and then glucosamine can be prepared by hydrolysis. Microbial fermentation has been widely used. In recent years, *Escherichia coli,* bacillus subtilis and fungi modified by metabolic engineering have been developed to produce glucosamine, but it has defects of low yield, high cost and complex refining process.

US 5 387 109 A discloses fructosylamine deglycase for decomposing amadori compounds of α-amino acids.

### SUMMARY

The objective of the disclosure is to provide a method for producing glucosamine and/or ketocarboxylic acid using a fructosamine deglycase enzyme, a transgenic cell line or a genetic engineering bacteria both expressing fructosamine deglycase. The method provided by the disclosure has sufficient substrate sources, which is not limited by raw materials, has mild preparing conditions, little environmental pollution, high reaction specificity, less impurities, which is easy for downstream separation and purification, and has low production cost and high glucosamine yield.

The present disclosure provides a method for producing glucosamine and/or ketocarboxylic acid, comprising:
performing an amino conversion by using an enzyme of fructosamine deglycase, or a transgenic cell line or a genetic engineering bacteria both expressing the fructosamine deglycase, and using amino acids and fructose-6-phosphate as substrates to obtain glucosamine and/or ketocarboxylic acid; the amino acid sequence of the fructosamine deglycase is set forth in SEQ ID NO:1.

In some embodiments, the amino acid comprises alanine, glutamic acid, aspartic acid or glutamine.

In some embodiments, the conditions of the amino conversion comprise: a pH value of 6-8 and a temperature of 30-50 °C.

In some embodiments, the enzyme in the method further comprises fructokinase, and an amino acid sequence of the fructokinase is set forth in SEQ ID NO:3.

The present disclosure further provides a method for co-producing glucosamine and ketocarboxylic acid by using a compound enzyme, comprising: mixing fructose and fructokinase for phosphorylation to obtain a fructose-6-phosphate; mixing the fructose-6-phosphate, amino acid and fructosamine deglycase for amino transfer to obtain glucosamine and ketocarboxylic acid, where the compound enzyme comprises fructosamine deglycase and fructokinase; an amino acid sequence of the fructosamine desugarase is set forth in SEQ ID NO:1; an amino acid sequence of the fructokinase is set forth in SEQ ID NO:3.

The present disclosure provides a method for catalyzing a transfer of amino from an amino donor compound to an amino receptor compound. The method provided by the disclosure has sufficient substrate sources, which is not limited by raw materials, has mild preparing conditions, little environmental pollution, high reaction specificity, less impurities, which is easy for downstream separation and purification, and has low production cost and high glucosamine yield. In a specific embodiment of the disclosure, gene sequence synthesis is conducted to the fructokinase (GenBank: ESD97983.1) derived from *Escherichia coli* 908658 and fructosamine deglycase (GenBank: AOR99552.1) derived from *Bacillus subtilis* by a gene synthesis method, and the resulting sequence is constructed in plasmid pCold II and stored in *Escherichia coli* JM109. According to the disclosure, the fructokinase protein and fructosamine deglycase protein are respectively obtained by successfully expressing *Escherichia coli* BL21 with high solubility, and the expressed biological enzyme protein is purified. According to the disclosure, the biological activity of the obtained recombinant fructokinase and recombinant fructosamine deglycase is verified, and it is found that fructose and fructose-6-phosphate can be synthesized into glucosamine by performing phosphorylation and transamination for the fructokinase and fructosamine deglycase in turn, and α-ketoglutarate can be generated by using glutamic acid at the same time, or pyruvic acid can be generated by transamination of alanine at the same time. Data show that fructokinase and fructosamine deglycase of the disclosure have good function of synthesizing glucosamine and pyruvic acid or α-ketoglutarate, and have great application potential. The method of the disclosure can greatly reduce the production cost of glucosamine and/or ketocarboxylic acid, amino acid and/or saccharide, be used as raw materials in pharmaceutical industry and food production industry, and has important significance.

### BRIEF DESCRIPTION OF THE DRAWINGS

Fig. 1 is a double digestion diagram of positive pCold II-*FrIB* plasmid provided by the present disclosure;
Fig. 2 is a double digestion diagram of positive pCold II-*FRK1* plasmid provided by the present disclosure;
Fig. 3 is an SDS-PAGE electrophoresis diagram of protein purification of fructokinase and fructosamine deglycase provided by the present disclosure.

### DETAILED DESCRIPTION OF THE EMBODIMENTS

A method for catalyzing a transfer of amino from an amino donor compound to an amino receptor compound is disclosed but not comprised entirely by the invention which is defined only by the appended claims, comprising: catalyzing the transfer of amino from an amino donor compound to an amino receptor compound by using an enzyme, or an expression vector or cloning vector expressing the enzyme, or a transgenic cell line and a genetic engineering bacteria expressing the enzyme; where the enzyme comprises:
1) an amino acid sequence set forth in SEQ ID NO:1: 
MSQATAKVNREVQAFLQDLKGKTIDHVFFVACGGSSAIMYPSKYVFDRESKSINSDLYSANE
FIQRNPVQLGEKSLVILCSHSGNTPETVKAAAFARGKGALTIAMTFKPESPLAQEAQYVAQY
DWGDEALAINTNYGVLYQIVFGTLQVLENNTKFQQAIEGLDQLQAVYEKALKQEADNAKQ
FAKAHEKESIIYTMASGANYGVAYSYSICILMEMQWIHSHAIHAGEYFHGPFEIIDESVPFIIL
LGLDETRPLEERALTFSKKYGKKLTVLDAASYDFTAIDDSVKGYLAPLVLNRVLRSYADELA
EERNHPLSHRRYMWKVEY;
2) an amino acid sequence formed by deletion, substitution, insertion or mutation of amino acids on the basis of the amino acid sequence set forth in SEQ ID NO:1, and having the enzyme activity that can catalyze the transfer of amino from an amino donor compound to an amino receptor compound.

The enzyme may be fructosamine deglycase (*FrIB*), a nucleotide sequence of the gene encoding the enzyme is set forth in SEQ ID NO:2: TTATATAACATTATAGTCTAATGCATAATGGTTCTTCATTTTCAGATCAATACTCAACTT TCCACATGTATCTTCTATGAGATAAAGGATGATTTCTCTCCTCTGCCAGCTCGTCTGCATA GCTTCTCAGCACACGATTGAGAACGAGCGGAGCAAGATAGCCTTTAACTGAATCGTCAAT TGCAGTGAAGTCGTAAGATGCAGCATCAAGCACAGTGAGCTTTTTGCCATACTTTTTCGA GAAGGTAAGCGCCCGCTCTTCAAGAGGTCTTGTTTCATCTAAACCGAGCAGGATGATAAA CGGCACGGATTCATCAATAATTTCAAACGGTCCGTGAAAATATTCTCCGGCATGAATGGC GTGGGAATGAATCCATTGCATTTCCATGAGAATGCAGATGCTGTAGGAGTAAGCGACACC GTAGTTTGCACCGCTTGCCATGGTATAAATAATACTTTCTTTTTCATGGGCTTTTGCAAAT TGCTTGGCGTTGTCAGCTTCCTGCTTAAGGGCTTTTTCATATACAGCCTGCAATTGATCTA AGCCTTCAATTGCTTGTTGGAATTTCGTATTGTTTTCTAATACTTGCAGGGTTCCAAAAA CGATTTGATACAAAACGCCATAGTTTGTATTGATCGCAAGCGCCTCATCACCCCAATCGTA CTGGGCAACATATTGCGCTTCCTGCGCTAAAGGAGACTCCGGTTTAAACGTCATCGCAATC GTAAGTGCACCCTTGCCCCTTGCAAACGCAGCAGCTTTGACTGTCTCCGGGGTATTTCCCG AATGCGAGCACAAAATAACAAGAGACTTTTCACCAAGCTGAACAGGGTTGCGCTGAATAA ATTCGTTGGCGCTGTAGAGGTCGGAGTTTATTGATTTTGACTCTCTGTCAAACACATACT TACTCGGATACATAATGGCAGAAGACCCTCCGCATGCGACAAAGAATACATGATCAATGG TTTTCCCTTTCAAATCCTGCAAGAAAGCTTGAACCTCACGATTTACTTTTGCTGTGGCCTG ACTCAAATCCTTCACTCCCCGTTTTTATTATATAACGTTATATAACATTATATAT. The fructosamine deglycase has the function of catalyzing the transfer of amino from an amino donor compound to an amino receptor compound. Transamination is conducted by the fructosamine deglycase of the present disclosure.

The amino donor compound includes amino acids. In some embodiments, the amino receptor compound preferably includes fructose and fructose-6-phosphate.

The method has the advantages of cheap raw materials, abundant sources, low production cost, environmental friendliness, safety to human bodies and the like.

Also provided is the use of fructosamine deglycase in the production of glucosamine and/or ketocarboxylic acid, and an amino acid sequence of the fructosamine deglycase is set forth in SEQ ID NO:1.

The invention provides a method for producing glucosamine and/or ketocarboxylic acid, comprising: performing an amino conversion by using an enzyme of fructosamine deglycase, or a transgenic cell line or a genetic engineering bacteria both expressing the fructosamine deglycase, and using amino acids and fructose and fructose-6-phosphate as substrates to obtain glucosamine and/or ketocarboxylic acid; an amino acid sequence of the fructosamine deglycase is set forth in SEQ ID NO:1, as further defined in the claims.

The saccharide includes fructose-6-phosphate. In the production process of the present invention, transamination is conducted by using fructose-6-phosphate and amino acids as substrates. The amino group of the amino acid is transferred to fructose-6 phosphate to obtain glucosamine, and the amino group is removed from the amino acid to obtain the ketocarboxylic acid. The starch, glucose and fructose of the present disclosure are preferably converted into fructose-6-phosphate before the above reaction is conducted. In some embodiments, the amino acids preferably include alanine, glutamic acid, aspartic acid or glutamine. After amino group transformation, each amino acid will generate corresponding ketocarboxylic acid. In some embodiments, the conditions for the amino conversion preferably include: pH 6-8, temperature 30-50 °C; more preferably pH 6.7-7.5, temperature 40-50 °C. In some embodiments, the enzyme used in the method preferably further comprises fructokinase, an amino acid sequence of the fructokinase is set forth in SEQ ID NO:3: MITN CRRPCIANPWRL Y AID IEKNKESTVRI GID LGGTKTEVIALGDAGEQL YRHRLPTPRD DYRQTIETIATLVDMAEQATGQRGTVGMGIPGSISPYTGWKNANSTWLNGQPFDKDLSAR LQREVRLANDANCLAVSEAVDGAAAGAQTVFAVIIGTGCGAGVAFNGRAHIGGNGTAGEW GHNPLPWMDEDELRYREEVPCYCGKQGCIETFISGTGFAMDYRRLSGHALKGSEIIRLVEES DPVAELALRRYELRLAKSLAHWNILDPDVIVLGGGMSNVDRLYQTVGQLIKQFVFGGECET PVRKAKHGDSSGVRGAAWLWPQE; a nucleotide sequence of the fructokinase is set forth in SEQ ID NO:4:
CTCTTGTGGCCATAACCACGCAGCGCCGCGTACGCCGCTGGAATCACCGTGCTTCGCCTTA CGCACCGGCGTTTCACATTCGCCGCCGAAGACAAATTGTTTAATCAACTGCCCAACCGTTT GATATAAACGGTCTACATTGCTCATCCCGCCCCCCAGGACAATCACATCCGGATCGAGAAT ATTCACGACATGTGCCAGCGATTTTGCCAGCCGCAGCTCGTAGCGACGCAATGCCAGTTCC GCTACCGGATCGCTTTCTTCAACCAGGCGGATAATTTCACTGCCTTTCAGCGCATGTCCGC TCAAACGACGATAATCCATCGCGAATCCCGTGCCCGAAATAAAGGTTTCAATACAACCTT GTTTACCGCAATAACAAGGGACTTCCTCGCGATAACGCAGTTCGTCTTCGTCCATCCACGG TAGCGGATTGTGTCCCCACTCACCTGCCGTGCCATTGCCGCCGATATGCGCCCGCCCATTG AATGCCACGCCCGCGCCGCATCCCGTGCCGATAATCACGGCAAATACCGTCTGCGCTCCCG CTGCCGCGCCATCTACTGCTTCTGAAACCGCCAGACAGTTAGCGTCATTTGCCAGCCGCAC TTCCCGCTGCAACCTCGCGCTTAAGTCTTTATCGAATGGCTGACCGTTGAGCCAGGTTGAA TTGGCATTCTTCACCACACCGGTGTAAGGCGAAATTGAGCCAGGAATGCCCATACCTACCG TTCCGCGCTGCCCCGTCGCCTGCTCCGCCATATCAACCAACGTGGCGATCGTTTCAATAGT CTGCCGGTAATCATCACGCGGCGTGGGCAGACGATGGCGGTACAACTGCTCCCCTGCATCG CCCAGTGCAATCACTTCAGTTTTGGTGCCGCCTAAATCGATACCTATACGCACGGTACTCT CCTTATTTTTTTCAATATCAATAGCGTAGAGACGGACAACCGGATTGGCAATGCAAGGCC GCCGACAATTCGTTATCAT. In some embodiments, when the saccharide is fructose, the catalytic system of the present disclosure preferably contains fructosamine deglycase and fructokinase, and the use of fructokinase can convert fructose into fructose-6-phosphate, and then carry out subsequent reaction above-mentioned.

Also disclosed but not comprised by the claimed scope is a vector of fructosamine deglycase, comprising a skeleton vector and a gene encoding fructosamine deglycase; and a nucleotide sequence of the gene encoding fructosamine deglycase is set forth in SEQ ID NO:2. The vector preferably includes an expression vector or a cloning vector, and when the vector is an expression vector, the skeleton vector preferably includes pCold II, pcold I or pUC19, and more preferably pCold II.

Further provided is a transgenic cell line for expressing fructosamine deglycase, and a nucleotide sequence of the gene encoding fructosamine deglycase is set forth in SEQ ID NO:2. There is no special limitation on the type and source of the cell line, and conventional commercial cell lines well known to those skilled in the art will do.

Further provided is a genetic engineering bacteria for expressing fructosamine deglycase, and a nucleotide sequence of the gene encoding fructosamine deglycase is set forth in SEQ ID NO:2. The host bacteria may include *Escherichia coli,* preferably include *Escherichia coli* BL21, *Escherichia coli* DH5α or *Escherichia coli* Top10, and more preferably *Escherichia coli* BL21.

A specific recombinant *Escherichia coli* genetic engineering bacteria may be constructed, and the constructing method preferably comprises:

The genes of fructokinase and fructosamine deglycase are searched through NCBI (National Center for Biotechnology Information) database. After finding the gene of the biological enzyme corresponding to the substrate, it is handed over to the biological company for gene synthesis, thus obtaining the above enzyme gene; the fructokinase gene is named *FRK1,* and the fructosamine deglycase gene is named *FrIB.*

Selecting pCold II as *E.coli* expression vector, recombinant expression vectors pCold *II-FRK1* and pCold *II-FrIB* are constructed, and the recombinant expression plasmids pCold II-*FRK1*and pCold *II-FrIB* are transformed into *E.coli* JM109, respectively, for preserving the recombinant expression vectors.

Further provided but not claimed is a method for producing fructosamine deglycase expressed by the genetic engineering bacteria, comprising: performing liquid culture and induction to the genetic engineering bacteria to obtain fructosamine deglycase.

After obtaining the recombinant expression vector, the method for producing fructosamine deglycase preferably comprises: positive recombinant *Escherichia coli* BL21/ pCold II-*FrIB* of the electrotransformation linearized recombinant expression plasmid pCold *II-FRK1* or pCold *II-FrIB* is cultured at 37 °C at 200 rpm until OD₆₀₀= 0.4-0.6; then the *Escherichia coli* BL21/ pCold *II-FrIB* is transferred to 15 °C for culture, added with isopropyl β-d-1-thiogalactopyranoside (IPTG) with a final concentration of 0.4 mM, and induced the expression for 24 h with a rotation speed of 200 rpm.

Further provided but not claimed is a compound enzyme for co-producing glucosamine and ketocarboxylic acid, where the compound enzyme comprises fructosamine deglycase and fructokinase; an amino acid sequence of the fructosamine desugarase is set forth in SEQ ID NO:1; an amino acid sequence of the fructokinase is set forth in SEQ ID NO:3.

The invention further provides a method for co-producing glucosamine and ketocarboxylic acid by using the compound enzyme above, comprising: mixing fructose and fructokinase for phosphorylation to obtain a fructose-6-phosphate; mixing the fructose-6-phosphate, amino acid and fructosamine deglycase for amino transfer to obtain glucosamine and ketocarboxylic acid, as further defined in the claims. In some embodiments, the amino acids preferably include alanine or glutamic acid. In some embodiments, when the amino acid is alanine, the ketocarboxylic acid is pyruvic acid; when the amino acid is glutamic acid, the ketocarboxylic acid is α-ketoglutaric acid. According to the method for co-producing glucosamine and ketocarboxylic acid, different types of ketocarboxylic acids can be prepared by changing the types of amino acids.

Specifically, the invention provides a method for co-producing glucosamine and pyruvic acid based on the compound enzyme described in the technical solution above, comprising: mixing fructose and fructokinase for phosphorylation to obtain a fructose-6-phosphate; mixing the fructose-6-phosphate, alanine and fructosamine deglycase and performing transamination to obtain glucosamine and pyruvic acid. The preparation method for co-producing glucosamine and pyruvic acid by enzymatic method not only meets the demand of low-cost glucosamine, but also meets the demand of quantity and price of pyruvic acid as raw material of health food. According to the disclosure, a biotransformation method, also known as a biocatalysis method, is utilized, and an extracted pure enzyme is used as a catalyst to complete transamination. At the same time, alanine is added as amino donor, which not only solves the need of amino catalyzed by enzyme, but also generates pyruvic acid as raw material of health food due to the transfer of amino in the preparation process of alanine. Glucosamine and pyruvic acid are produced with high yield and low cost through a biotransformation method. According to the method, the production efficiency is improved by controlling the addition amount of enzymes, and the generation of byproducts is reduced; two products of glucosamine and pyruvic acid are obtained through simple separation, thus greatly reducing the prices of the two products.

The disclosure provides a method for co-producing amino acid and α-ketoglutarate based on the compound enzyme in the technical solution above, comprising: mixing fructose and fructokinase for phosphorylation to obtain a fructose-6-phosphate; mixing the fructose-6-phosphate, glutamic acid and fructosamine deglycase and performing transamination to obtain glucosamine and α-ketoglutarate. According to the disclosure, glutamic acid is added, and the amino group for producing glucosamine is provided by glutamic acid; in the process of enzymatic preparation, not only is glucosamine with high concentration and high purity prepared, but also glutamic acid is transformed into α-ketoglutaric acid with high added value. Therefore, glucosamine and α-ketoglutarate are prepared by enzymatic co-production. The method of the present disclosure has the advantages of low production cost, high product purity, simple preparation steps and the like.

The fructosamine deglycase vector, transgenic cell line and a genetic engineering bacteria expressing fructosamine deglycase, and use of fructosamine deglycase according to the present disclosure will be further explained in detail with specific examples below.

Terms used in the present disclosure, unless otherwise specified, generally have meanings commonly understood by those of ordinary skills in the art.

In the following examples, various processes and methods not described in detail are conventional methods known in the art.

### EXAMPLE 1

### Acquisition of fructokinase and fructosamine deglycase genes

At first, the genes of fructokinase and fructosamine deglycase were searched through NCBI (National Center for Biotechnology Information) database. The fructokinase from *Escherichia coli* 908658 (GenBank: ESD97983.1) and fructosamine deglycase from Bacillus subtilis (GenBank: AOR99552.1) were obtained by gene mining, and then they were handed over to biological company for gene synthesis. In which the *SnaBI* and *NotI* restriction sites (without signal peptide) were introduced into the synthesized gene sequence, the restriction sites were protective bases, and the effective sequence was the sequence after the restriction site. And the synthesized gene sequence was stored in the plasmid pMD-19T to form a recombinant plasmid pMD-19T-*FRK1* or pMD-19T-*FrIB*.

Transformation was conducted to the synthesized gene sequence, that is, 10 µl of ligation product was taken and added into competent cells JM109, then put on ice for 30 min, immediately put on ice after being heated at 42 °C for 90 s, then put on ice for 2 min, 1ml LB culture medium without antibiotics was added, and it was cultured on a shaking table at 37 °C for 1h, then the transformed bacterial solution was coated on LB(Amp) blue whiteboard and cultured overnight at 37 °C. Several colonies were selected from the plate for PCR identification.

At the same time, plasmid extraction was used, and clone plasmid identified as positive by PCR was extracted by plasmid extraction kit of OMEGA Company.

### EXAMPLE 2

Construction of prokaryotic expression vectors, recombinant expression and protein expression of fructokinase and fructosamine deglycase genes
1. Construction of prokaryotic expression vector
   (1) Primer design: Primers were designed from the mature peptide sequence after the signal peptide.
      Primers of fructosamine deglycase:
      Forward primer:
         5'-TACGTAAATATATTGTAATATCAGATTACGT-3' (SEQ ID NO:5);
      Reverse primer:
         5'-GCGGCCGCGTTATATAACATTATAGTCTAATGCA-3' (SEQ ID NO:6);
      The underlined part was the restriction site, the enzymes used were SnaB I and Not **I**.
      Fructokinase primer:
         Forward primer:
         5'-TACGTAGAGAACACCGGTATTGGTGCGTCGC-3' (SEQ ID NO:7);
      Reverse primer:
         5'-GCGGCCGCGCTCTTGTGGCCATAACCACGCAGCG-3' (SEQ ID NO:8);
      The underlined part was the restriction site, the enzymes used were SnaB I and Not **I.**
   (2)PCR reaction was conducted, cloning vector pMD-19T-*FRK1* or pMD-19**T**-*FrIB* were used as template, annealing at 62 °C for 35 cycles.
   (3) *SnaB I* and *Not I* double digestion of the PCR product of the corresponding biological enzyme gene and plasmid pCold II. The enzyme digestion system is shown in Table 1.

**Table 1 Double enzyme digestion system**

| Component | Amount |
|---|---|
| Purified PCR product/Plasmid | 30µl |
| 10*quitcut buffer | 5 µl |
| QuitCut SnaB I | 1µl |
| QuitCut Not I | 1µl |
| ddHzO | 13µl |
| Total volume | 50µl |

The system was digested at 37 °C for 2 hours.

(4) According to the conventional method, it was ligated, transformed and identified by double enzyme digestion.

The clone extraction plasmid was sequenced at both ends of AOX3 and AOX5, which further verified the correctness of the inserted target gene.
2. Expression of recombinant plasmid in *Escherichia coli* BL21
1) Screening of transformed *Escherichia coli* and positive transformants

According to the operation manual of *E.coli* expression system, 10 µl of ligation product was taken and added into competent cells DE3, then put on ice for 30 min, immediately put on ice after being heated at 42 °C for 90 s, then put on ice for 2 min, 1ml LB culture medium without antibiotics was added, and then it was cultured in on shaking table at 37 °C for 1h, then the transformed bacterial solution was coated on LB(Amp) blue whiteboard and cultured overnight at 37 °C. Several colonies were selected from the plate for PCR identification. Compared with the bacteria transformed by empty vector, it was further verified that the target gene had been transferred into *E.coli* BL21.

### 2) Induced expression of recombinant plasmid in Escherichia coli

The recombinant *Escherichia coli* was cultured in 25-50 mL LB medium (250 mL triangular flask) until the OD value reached 0.4-0.6, and then transferred to 15 °C for culture, and added with Isopropyl β-d-1-thiogalactopyranoside (IPTG) with a final concentration of 0.4 mM, and an induced expression was conducted for 24 h at a rotation speed of 200 rpm. Then, the bacteria were broken by ultrasonic wave and the recombinant enzyme was extracted. Meanwhile, SDS-PAGE and enzyme activity of the culture solution were determined.

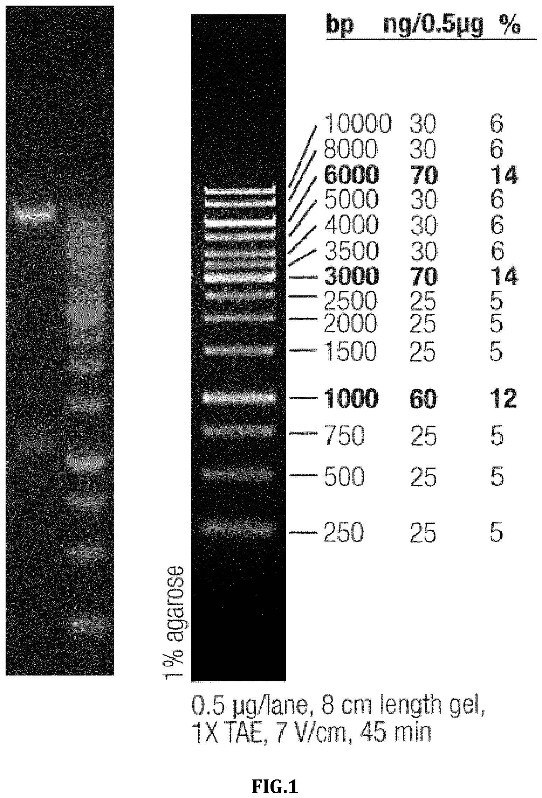

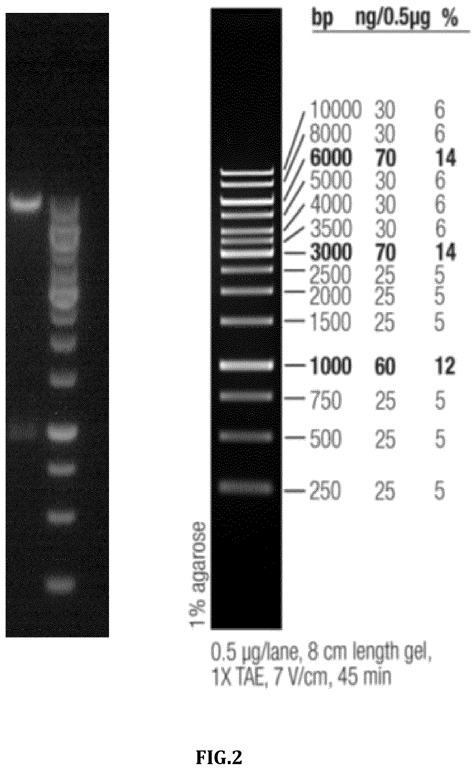

Fig. 1 is a double digestion diagram of positive pCold *II-FrIB* plasmid, M is a Marker of 10000bp; Fig. 2 is a double digestion diagram of positive pCold II-*FRK1* plasmid, M is a Marker of 10000bp. The two diagrams have obvious bands around 1082bp and 993bp respectively, which is consistent with the theoretical values, thus proving that the expression vectors of the two genes have been successfully constructed.

### EXAMPLE 3

### Purification of fructokinase and fructosamine deglycase protein

Purification of recombinant enzyme and SDS-PAGE gel electrophoresis analysis thereof.

Protein was purified according to GE Healthcare guidelines, and SDS-PAGE analysis was carried out according to Molecular Cloning Experimental Guidelines (Third Edition). The gel concentration used was 12.5%, and the sample loading was 5-25µl. Protein was stained with Coomassie Brilliant Blue R-250.

The experimental steps of native-SDS-PAGE include:
A. 5-10µl of sample buffer ((0.1 mol/L Tris-HC1, pH 6.8), 2 %SDS (weight: volume), 10% glycerol (volume: volume), and 0.01% bromophenol blue (weight: volume)) were added into 5-10µl enzyme solution and then placed in a water bath at 37 °C for 5-10 min, and then separation was conducted by electrophoresis. Note: when extracting samples, the reason why mercaptoethanol is not added in the sample extract is to denature proteases moderately during electrophoresis, so as to restore the activity of these proteases after electrophoresis. β-mercaptoethanol was used to open disulfide bonds, so as to destroy the quaternary or tertiary structure of protein. It is a colorless and transparent liquid with special odor, which is flammable and easily soluble in water, alcohol, ether and other organic solvents.
B. Gel preparation and electrophoresis: 0.2% gelatin was added in the process of preparing a separation gel, and then gel filling was carried out after being evenly mixed, and Gelatin-SDS-PAGE (substrate gel) was obtained after solidification. The density of polyacrylamide in "concentrated gel" was 5%, and that in "separated gel" was 12%, with a thickness of 1mm³. Running electrophoresis by loading. Note: Gelatin was added during gel preparation, so it has been crosslinked in the gel, and will not migrate under the action of electric field during electrophoresis.
C. SDS removal: after electrophoresis, the gel was soaked in renaturation buffer (2% TritonX-100, 50 mmol/LTris-HC1, pH 7.5) for 2-3 times, each time for 5-10 min.
D. renaturation: the separation gel was placed in a buffer solution (50 mmol/L Tris-HC1, pH7.5) at 37 °C for enzyme reaction for 3 hours.
E. dyeing and decolorization: coomassie brilliant blue was used for dyeing for 30 min, and then the decolorizing solution (5% acetic acid +10% methanol) was changed for several hours until the background was clear. Note: the background color of the gel after dyeing and decolorization is blue-black, and the color of protease reaction site becomes lighter. The size of the region where protease reaction occurs in the gel and the light transmittance of this region are directly proportional to protease activity.

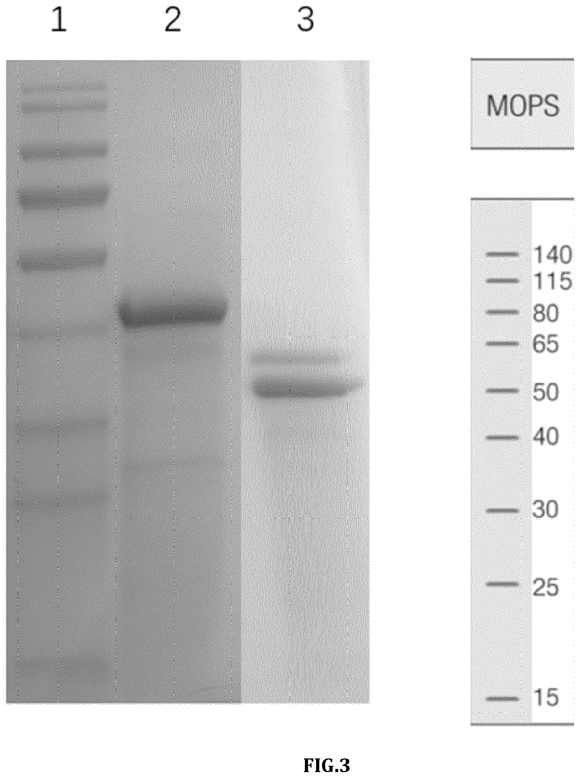

Fig. 3 is an SDS-PAGE electrophoretogram of protein purification of fructokinase and fructosamine deglycase. As shown in Fig. 3, lane 1 is a protein Marker; lane 2 is the SDS-PAGE electrophoresis of fructosamine deiminase *FrIB;* lane 3 is the SDS-PAGE electrophoresis of fructokinase *FRK1.* The two proteins have obvious bands around 39.7kDa (fructosamine deglycase) and 36.4kDa (fructokinase), which are consistent with the theoretical values, thus proving that both proteins have been successfully expressed and purified.

### EXAMPLE 4

### Activity detection of purified fructokinase and fructosamine deglycase

### 1. Detection of fructokinase activity

Enzyme activity was determined by enzyme-linked assay. The 400µL reaction solution included 30 mmol/L Hepes-NaOH(pH 7.5), 1 mmol/L MgClz, 0.6 mmol/L Na₂EDTA, 9 mmol/L KCl, 1 mmol/L NAD, 1 mmol/L ATP, 2 mmol/L fructose. 1 U Glc-6-P DH (from Leuconost °C mesotriodes), 1 U phosphoglucoisozyme (PGI, Sigma), after adding 80µL enzyme extract at 25 °C, the reaction started, and the absorption value at 340 nm was measured. The activity of fructokinase was measured as (NAD+ yield)/ min, that was, the change value of NAD+ absorbance.

### 2. Detection of fructosamine deglycase activity

### Reaction system:

1mL reaction system contained 15mM glutamic acid, 20mM fructose-6-pHosphate, 0.2 mL fructosamine deglycase, 2.5mM EDTA and 100mM buffers with different pH values (Na₂HPO₄-NaH₂PO₄, pH2.0-10.0). The above reaction systems was put into a 1.5mL centrifuge tube, mixed evenly, and then put into PCR to react at 37 °C for 20min, then the reaction was terminated by heating at 95 °C for 5min. After centrifugation, the supernatant was taken to detect the production of α-ketoglutarate by high performance liquid chromatography (HPLC) to calculate the fructosamine desaccharase activity, so as to calculate the activity of fructosamine deglycase.

### Testing conditions:

The α-ketoglutarate was quantitatively analyzed by high performance liquid chromatography (HPLC). A Hypersil BDS column was used, the mobile phase was 0.1 mol·L⁻¹ (NH₄)H₂PO₄, with a pH of 2.65, the flow rate was 1.0 mL/min, the column temperature was 40 °C, and the detection wavelength was 215 nm. The content of α-ketoglutaric acid in the reaction solution was calculated by peak area according to the external standard method.

### Enzyme activity definition:

Under the optimum reaction temperature of 37 °C, the amount of enzyme needed to catalyze the conversion of 1µmol glutamic acid to α-ketoglutarate per minute was defined as one enzyme activity unit, namely 1U.

The enzyme activity of fructokinase was determined to be 1.6221U; the activity of fructosamine deglycase was 2.7859U.

### EXAMPLE 5

### Synthesis reaction system for co-producing glucosamine and α -ketoglutarate

### Reaction system:

1mM fructose; 100mM HEPES buffer (pH 7.0); 10mM MgCl; 15mM glutamic acid; 2.5mM EDTA; 10U fructosamine deglycase; 10U fructokinase. This reaction system was reacted for 1 hour and then used for detection. Through quantitative analysis of glucosamine, the reaction process was controlled. While the reaction produced glucosamine, glutamic acid was converted into α-ketoglutarate due to transamination.

### Testing conditions:

Glucosamine was quantitatively analyzed by high performance liquid chromatography (HPLC). The chromatographic column was amino column, the mobile phase was 80% acetonitrile aqueous solution, the flow rate was 0.6mL/min, the column temperature was 40 °C, and the detector was differential refraction detector. The retention time of glucosamine was about 12.7 minutes. The concentration of glucosamine was proportional to the response intensity of HPLC characteristic peak of glucosamine.

After the above experimental process, it was detected that the glucosamine produced after the reaction for 1 hour was 0.65 mM.

### EXAMPLE 6

### Synthesis reaction system of glucosamine and pyruvic acid

### Reaction system:

1mM fructose; 100mM HEPES buffer (pH 7.0); 10mM MgCl; 15mM alanine; 2.5mM EDTA; 10U fructosamine deglycase; 10U fructokinase. This reaction system was reacted for 1 hour and then used for detection. Through quantitative analysis of glucosamine, the end point of the synthetic reaction was determined. Finally, glucosamine was prepared by separation, and pyruvic acid, a health food raw material with natural characteristics, was also prepared.

### Testing conditions:

Glucosamine was quantitatively analyzed by high performance liquid chromatography (HPLC). The chromatographic column was amino column, the mobile phase was 80% acetonitrile aqueous solution, the flow rate was 0.6mL/min, the column temperature was 40 °C, and the detector was differential refraction detector. The retention time of glucosamine was about 12.7 minutes. The concentration of glucosamine was proportional to the response intensity of HPLC characteristic peak of glucosamine.

After the above experimental process, it was detected that the glucosamine produced after the reaction for 1 hour was 0.78 mM.

## Claims

1. A method for producing glucosamine and/or ketocarboxylic acid, comprising:
performing an amino conversion by using an enzyme of fructosamine deglycase, or a transgenic cell line or a genetic engineering bacteria both expressing the fructosamine deglycase, and using amino acids and fructose-6-phosphate as substrates to obtain glucosamine and/or ketocarboxylic acid; the amino acid sequence of the fructosamine deglycase is set forth in SEQ ID NO:1.

2. The method according to claim 1, wherein the amino acid comprises alanine, glutamic acid, aspartic acid or glutamine.

3. The method according to claim 1, wherein the conditions of the amino conversion comprise: a pH value of 6-8 and a temperature of 30-50 °C.

4. The method according to claim 1, wherein the method further comprise to use fructokinase, the amino acid sequence of the fructokinase is set forth in SEQ ID NO:3.

5. A method for co-producing glucosamine and ketocarboxylic acid by using the compound enzyme, comprising: mixing fructose and fructokinase for phosphorylation to obtain a fructose-6-phosphate; mixing the fructose-6-phosphate, amino acid and fructosamine deglycase for amino transfer to obtain glucosamine and ketocarboxylic acid, wherein the compound enzyme comprises fructosamine deglycase and fructokinase; the amino acid sequence of the fructosamine desugarase is set forth in SEQ ID NO: 1; the amino acid sequence of the fructokinase is set forth in SEQ ID NO:3.

## Patentansprüche

1. Verfahren zur Produktion von Glucosamin und/oder Ketocarbonsäure, umfassend:
Durchführen einer Aminoumwandlung durch Verwenden eines Enzyms von Fructosamindeglycase oder einer transgenen Zelllinie oder einer gentechnischen Bakterie, die beide Fructosamindeglycase exprimieren, und Verwenden von Aminosäuren und Fructose-6-phosphat als Substrate, um Glucosamin und/oder Ketocarbonsäure zu erhalten; wobei die Aminosäuresequenz der Fructosamindeglycase in SEQ ID Nr. 1 dargelegt ist.

2. Verfahren nach Anspruch 1, wobei die Aminosäure Alanin, Glutaminsäure, Asparaginsäure oder Glutamin umfasst.

3. Verfahren nach Anspruch 1, wobei die Bedingungen der Aminoumwandlung umfassen: einen pH-Wert von 6-8 und eine Temperatur von 30-50°C.

4. Verfahren nach Anspruch 1, wobei das Verfahren ferner ein Verwenden von Fructokinase umfasst, wobei die Aminosäuresequenz der Fructokinase in SEQ ID Nr. 3 dargelegt ist.

5. Verfahren zur Koproduktion von Glucosamin und Ketocarbonsäure durch Verwendung des Verbindungsenzyms, umfassend: Mischen von Fructose und Fructokinase zur Phosphorylierung, um ein Fructose-6-phosphat zu erhalten; Mischen von Fructose-6-phosphat, Aminosäure und Fructosamindeglycase zum Aminotransfer, um Glucosamin und Ketocarbonsäure zu erhalten, wobei das Verbindungsenzym Fructosamindeglycase und Fructokinase umfasst; wobei die Aminosäuresequenz der Fructosamindeglycase in SEQ ID Nr. 1 dargelegt ist; wobei die Aminosäuresequenz der Fructokinase in SEQ ID Nr. 3 dargelegt ist.

## Revendications

1. Procédé de production de glucosamine et/ou d'acide cétocarboxylique, comprenant :
la réalisation d'un conversion amino en utilisant une enzyme de fructosamine déglycase, ou une lignée cellulaire transgénique ou une bactérie manipulée génétiquement exprimant toutes deux la fructosamine déglycase, et l'utilisation des acides aminés et du fructose-6-phosphate comme substrats afin d'obtenir de la glucosamine et/ou de l'acide cétocarboxylique ; la séquence d'acides aminés de la fructosamine déglycase étant exposée dans la SEQ ID NO:1.

2. Procédé selon la revendication 1, dans lequel l'acide aminé comprend l'alanine, l'acide glutamique, l'acide aspartique ou la glutamine.

3. Procédé selon la revendication 1, dans lequel les conditions de la conversion amino comprennent : une valeur de pH de 6 à 8 et une température de 30 à 50 °C.

4. Procédé selon la revendication 1, dans lequel le procédé comprend en outre l'utilisation de fructokinase, la séquence d'acides aminés de la fructokinase étant exposée dans la SEQ ID NO:3.

5. Procédé de co-production de glucosamine et d'acide cétocarboxylique en utilisant l'enzyme composite, comprenant : le mélange du fructose et de la fructokinase pour la phosphorylation afin d'obtenir un fructose-6-phosphate ; le mélange du fructose-6-phosphate, de l'acide aminé et de la fructosamine déglycase pour le transfert amino afin d'obtenir de la glucosamine et de l'acide cétocarboxylique, dans lequel l'enzyme composite comprend de la fructosamine déglycase et de la fructokinase ; la séquence d'acides aminés de la fructosamine désugarase étant exposée dans la SEQ ID NO:1 ; la séquence d'acide aminé de la fructokinase étant exposée dans la SEQ ID NO: 3 .
